# EUROPEAN PATENT APPLICATION

(11) **EP 4 458 359 A1**
(43) Date of publication of application: **06.11.2024**
(21) Application number: 23795414.4
(22) Date of filing: 25.04.2023
(51) Int. Cl.: A61K 31/473, A61P 35/00

(54) **PHARMACEUTICAL COMPOSITION OF ETHIDIUM BROMIDE, AND USE THEREOF IN TREATING CANCER**

(30) Priority: 29.04.2022 CN 202210475952
(71) Applicant: Ruijin Hospital, Shanghai Jiaotong University School of Medicine, Shanghai 200025 (CN); Shanghai Institute for Endocrine and Metabolic Diseases, Shanghai 200025 (CN)
(72) Inventor: HUANG, Guorui, Shanghai 200025 (CN); ZHANG, Chen, Shanghai 200025 (CN); NING, Guang, Shanghai 200025 (CN); WANG, Weiqing, Shanghai 200025 (CN)
(74) Representative: HGF
(86) International application number: PCT/CN2023/090596
(87) International publication number: WO 2023/207982

(57) **Abstract**

The present invention relates to a pharmaceutical composition of ethidium bromide and, in particular, to its potential use as a drug for the treatment of cancer. The present invention demonstrates that ethidium bromide has good targeting effect on cancer cells, selectively induces apoptosis in cancer cells and effectively inhibits tumor migration. At the same time ethidium bromide has little or almost no side effects. This indicates that ethidium bromide has high clinical application value, and is expected to promote clinical trials and its application.

## Description

### Technical Field

The present invention is in the field of anti-tumor drugs and relates to a pharmaceutical composition of ethidium bromide and, in particular, to the potential use of ethidium bromide in the preparation of a drug for the treatment of cancer.

### Background Art

Cancer is a disease caused by multiple factors. The current prevailing view of carcinogenesis is that it is a multi-step process controlled by multiple genes, which is similar to Darwin's theory of evolution. But based on them it is difficult to find effective methods and strategies to treat cancer. Likewise, another hypothesis regarding tumorigenesis, the abnormal glycolysis hypothesis of carcinogenesis, known as the "Warburg effect", was awarded the Nobel Prize in 1923.

For many years, the most important and widespread method of tumor diagnosis has still been PET-CT, the most important theoretical basis for which is the "Warburg effect". It is generally believed to be a tumor diagnostic technique developed using the characteristics of tumor tissue that have a high demand for energy to cope with its rapid proliferation, resulting in high glucose uptake. However, a contradiction arises in this process: why do tumor cells that require more energy instead choose an inefficient glycolytic pathway to consume glucose and gain energy? Undoubtedly, clarifying this paradoxical metabolic process in tumor cells will not only contribute to an in-depth understanding of tumor properties and provide better clinical diagnostics but will also expand anti-tumor strategies and potentially lead to the screening of more potent and broad-spectrum anti-tumor drugs.

Although only 5% of glucose in cancer cells is thought to produce ATP via the mitochondrial pathway, it accounts for more than half of the energy source. Given that cancer cells have a higher energy requirement and are more sensitive to changes in energy supply, it is possible that they are more sensitive to drugs that target mitochondrial function. Therefore, further inhibition of the already dysfunctional mitochondria is likely to be the "last straw that breaks the back of tumor cells".

Ethidium bromide is an organic compound that can induce mitochondrial damage and has the following structural formula.

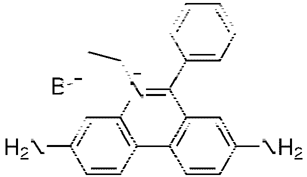

Ethidium bromide is commonly used as a nucleic acid dye for nucleic acid staining, and its tricyclic planar moiety can be embedded between DNA stacking bases in a non-base sequence-specific manner. Ethidium bromide is generally considered to be a mutagen with carcinogenicity. However, according to the Pharmacopeia of Compounds edited by the FDA, US, there is no evidence that ethidium bromide can induce DNA mutations and carcinogenesis in cells or organisms. (National Toxicology Program, 1239-45-8). In contrast, studies have shown that cells treated with long-term ethidium bromide do not create genetic mutations (Mutation Research, 174(1986) 175-178).

The present invention surprisingly found that ethidium bromide has excellent anti-tumor effects and thus has very high clinical application value.

### Summary of the Invention

In one aspect, provided by the present invention is a pharmaceutical composition comprising a therapeutically effective amount of ethidium bromide, or a pharmaceutically acceptable salt, hydrate, solvate, or prodrug thereof, and a pharmaceutically acceptable carrier.

In another aspect, provided by the present invention is use of ethidium bromide, or a pharmaceutically acceptable salt, hydrate, solvate, or prodrug thereof, in the preparation of a pharmaceutical composition.

In another aspect, provided by the present invention is a method for treating cancer, comprising administering to a subject in need thereof the aforementioned pharmaceutical composition.

In another aspect, provided by the present invention is a method for inhibiting tumor metastasis, comprising administering to a subject in need thereof the aforementioned pharmaceutical composition.

In another aspect, provided by the present invention is a method for activating an mTOR signaling pathway, comprising administering to a subject in need thereof the aforementioned pharmaceutical composition.

In another aspect, provided by the present invention is a method for selectively inducing apoptosis in cancer cells, comprising contacting said cancer cells with ethidium bromide, or a pharmaceutically acceptable salt, hydrate, solvate, or prodrug thereof.

The present invention surprisingly found that ethidium bromide has good targeting effects on cancer cells, can selectively induce apoptosis in cancer cells, and effectively inhibit tumor migration. It has been proved that ethidium bromide can effectively clear tumors *in vivo,* such as colorectal tumor tissue, and has a very good inhibitory effect on the proliferation and migration of cancer cells, such as the migration of pancreatic cancer cells in pancreas, liver and lung tissues.

It is even more difficult and surprising that ethidium bromide has little or almost no side effects, which is in contrast to the general knowledge of the prior art. Therefore, ethidium bromide has extremely high clinical application value and is expected to promote clinical trials and its application.

### Brief Description of the Drawings

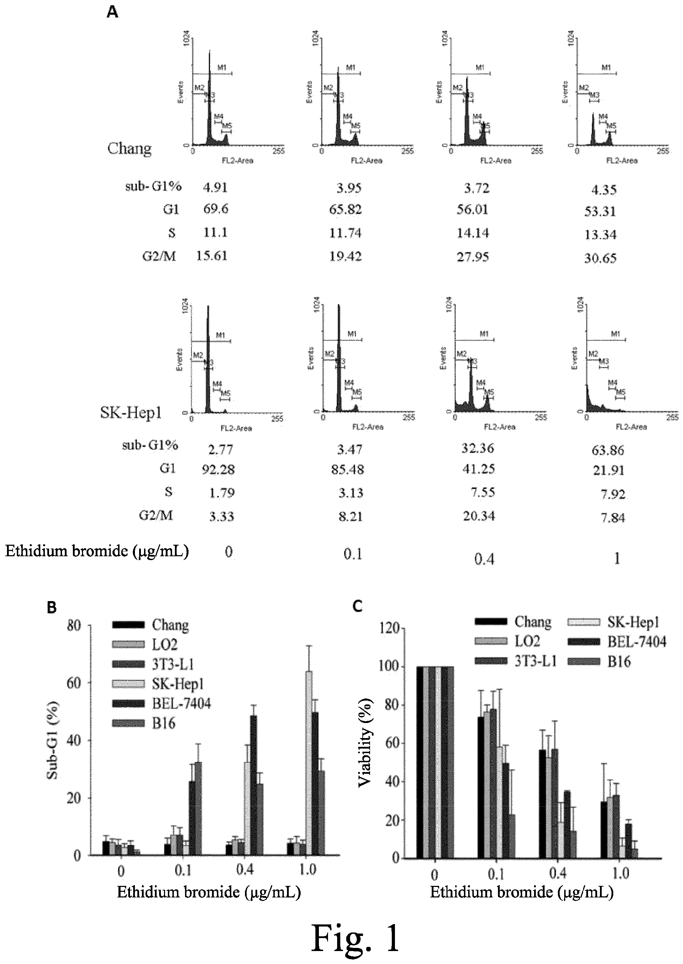

FIG. 1 shows that ethidium bromide (RJ101) can selectively induce death of various liver cancer and melanoma cells. (A) Detection and analysis results of cell cycles of normal liver cell Chang and hepatocellular carcinoma cell SK-Hep1 by flow cytometry. (B) Proportion of cells containing cleaved DNA (Sub-G1), i.e. the proportion of apoptosis, by flow cytometry after the cells were treated with different concentrations of drugs, wherein Chang, LO2, and 3T3-L1 were normal cell lines, and the other three were cancer cell lines. (C) Cell viability measured by the MTT method with values representing the relative number of viable cells in ethidium bromide-treated cells to untreated control group, respectively.

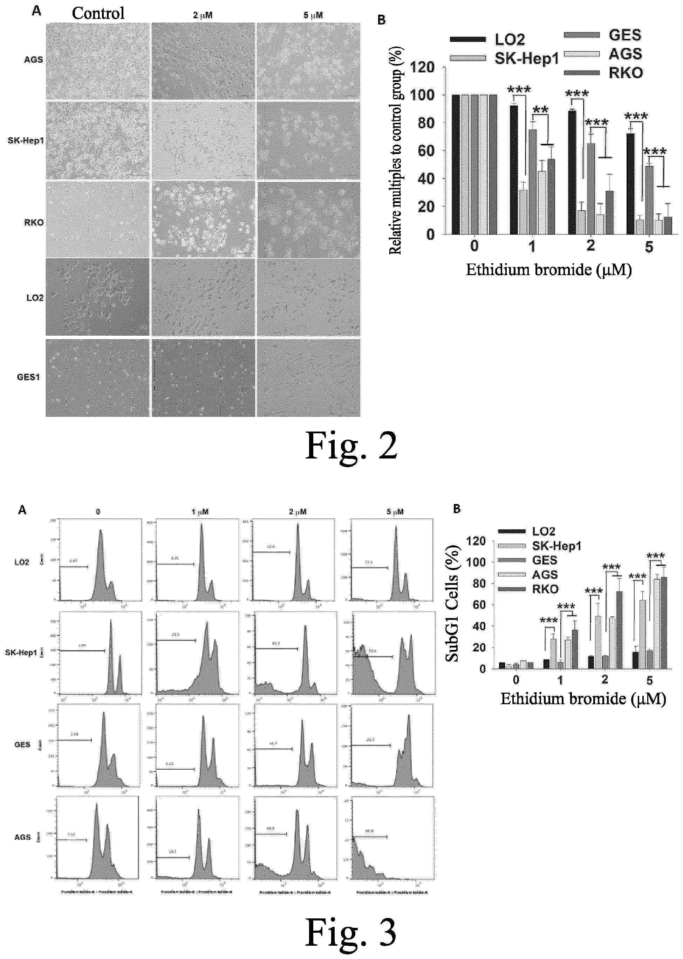

FIG. 2 shows that ethidium bromide can selectively induce the death of gastric cancer cells (AGS), hepatocellular carcinoma cells (SK-Hep1), and colorectal cancer cells (RKO). (A) Representations of the cell survival status taken under an optical microscope after the cells were treated with different concentrations of drugs. (B) Cell viability measured by MTT after the cells were treated with different concentrations of drugs.
FIG. 3 shows that ethidium bromide can selectively induce the death of gastric cancer cells (AGS), hepatocellular carcinoma cells (SK-Hep1), and colorectal cancer cells (RKO). (A) Results of cell cycle analysis by flow cytometry, wherein GES1 (stomach) and LO2 (liver) were non-cancer cell lines. (B) Statistical analysis of Sub-G1 cells in the flow cytometry results.

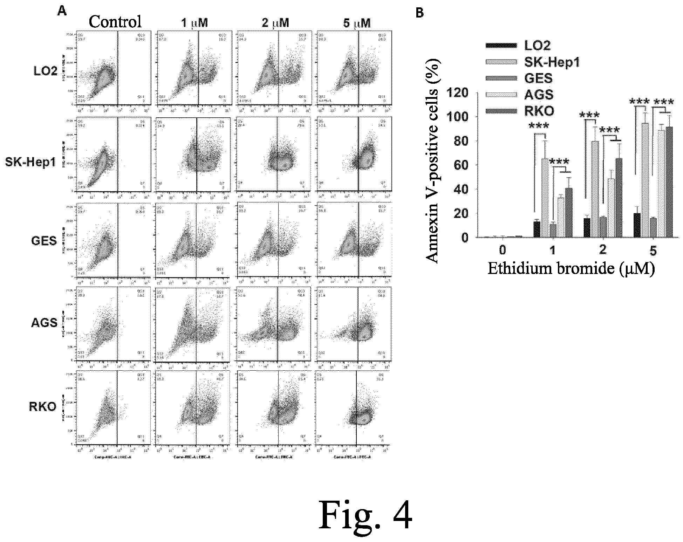

FIG. 4 shows that ethidium bromide can selectively induce apoptosis in gastric cancer cells (AGS), hepatocellular carcinoma cells (SK-Hep1), and colorectal cancer cells (RKO). (A) Apoptosis detected by Annexin V staining. (B) Analytical statistics for Annexin V positive.

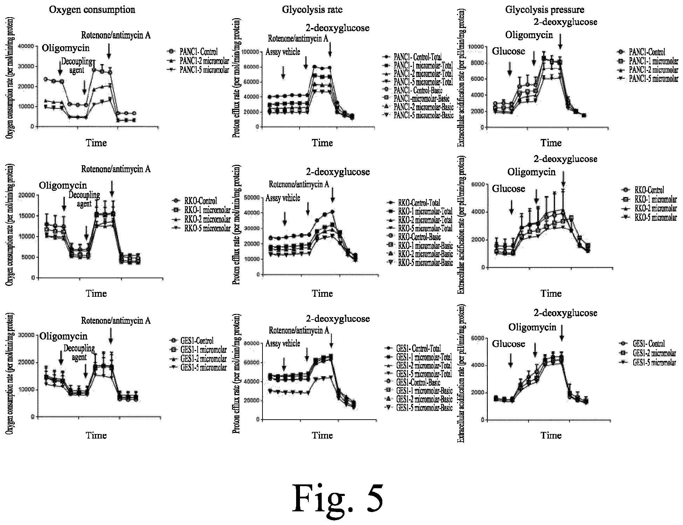

FIG. 5 shows the effect of ethidium bromide on oxygen consumption, glycolysis levels, and glycolysis rate of cells. Effects of ethidium bromide on oxygen consumption (A, D, and G), glycolytic levels (B, E, and H), and glycolytic rates (C, F, and I) of human pancreatic cancer cells Panc1 (A-C), colorectal cancer cells RKO (D-F) and normal gastric fibroblasts GES1 (G-I).

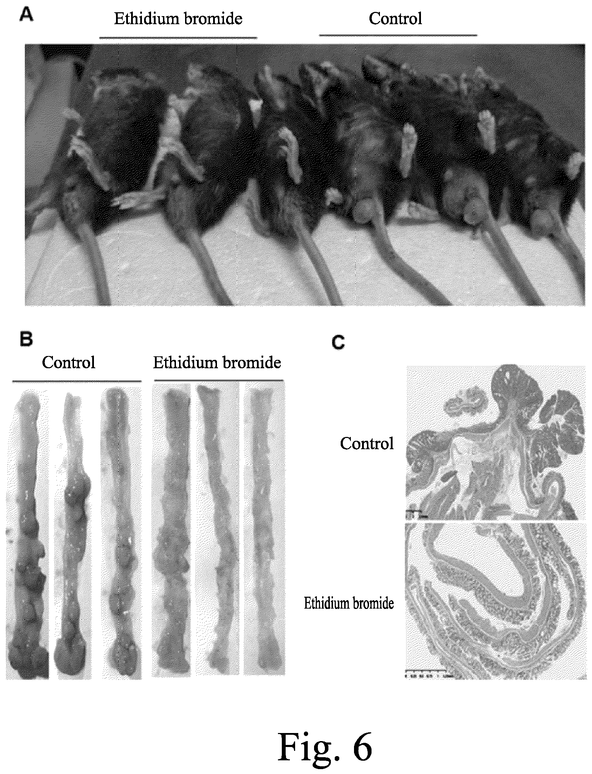

FIG. 6 shows the effect of ethidium bromide (orally administered via drinking water) on AOM + DSS-induced colorectal tumor activity. (A) Appearance of mice in the dosing and control groups. (B) Representative colorectal images of mice in the dosing and the control groups. (C) H & E staining of colorectal samples from mice in the dosing and the control groups (Vehicle).

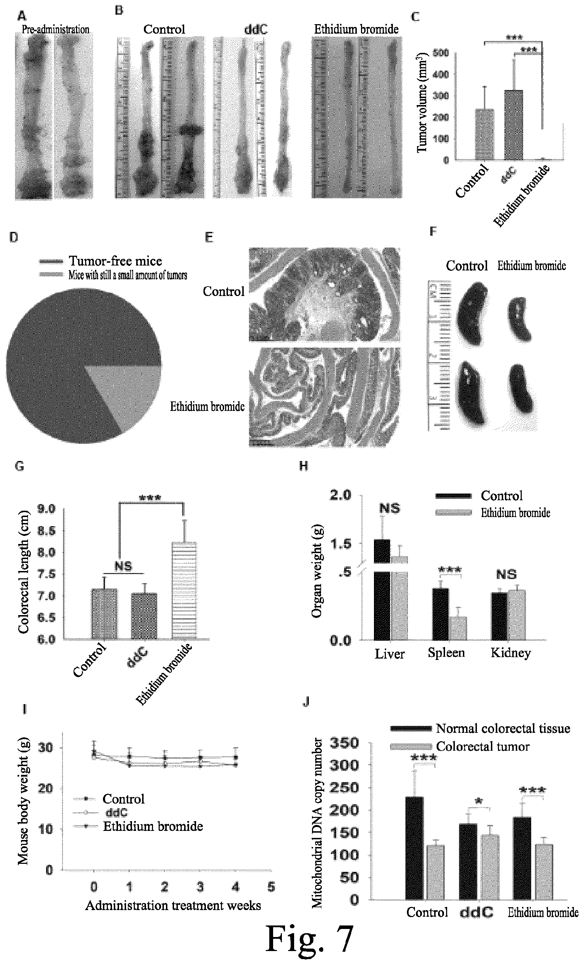

FIG. 7 shows the anti-tumor activity of ethidium bromide (gavage administration) on AOM + DSS-induced colorectal cancer mice. (A) Tumor growth in mice after induction and pre-administration. (B) Tumors in mice after one month in the control group, the dosing group (ddC and ethidium bromide), a total of three groups. (C) Tumor volumes of different groups. (D) Proportions of mice with or without complete tumor clearance, respectively. (E) Representative H & E staining of colorectal samples from mice in the dosing and the control groups. (F) Representative spleen size of mice in the dosing and the control groups. (G) Statistical analysis of colorectal length of mice in different treatment groups. (H) Liver, spleen, and kidney weights in the dosing and control groups. (I) Body weights of mice in different treatment groups. (J) Mitochondrial DNA (mtDNA) copy number of normal colorectal tissue and tumor tissue in different treatment groups.

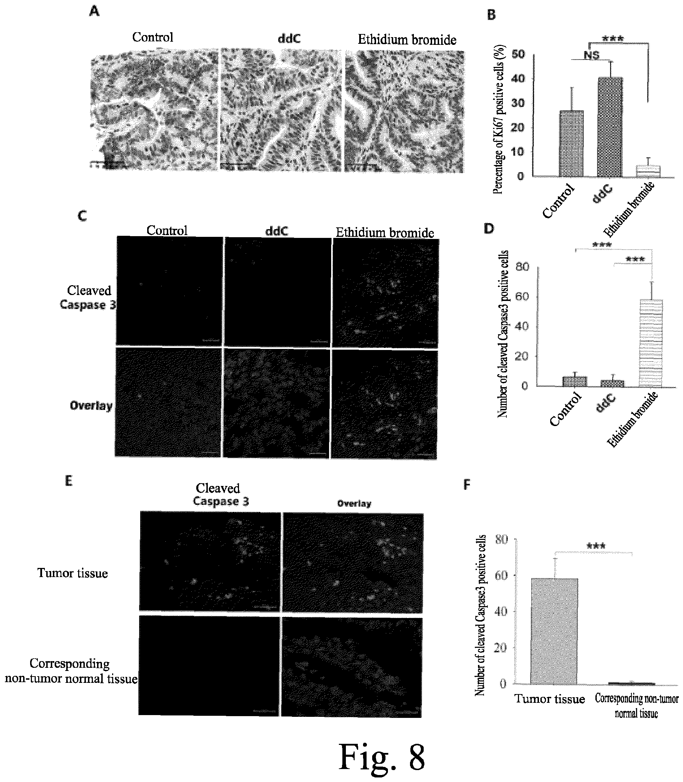

FIG. 8 shows that ethidium bromide can inhibit tumor cell proliferation and induce tumor cell apoptosis. (A) Proliferation of tumor cells in different treatment groups detected by Ki67. (B) Proportion of Ki67 positive cells by quantitative analysis. (C) Cleaved Caspase3 positive cells in different treatment groups detected by immunofluorescence. (D) Quantification of results in (C). (E) Difference in cleaved Caspase3 positive cells in the tumor and normal tissues surrounding the tumor detected by immunofluorescence. (F) Quantification of results in (E).

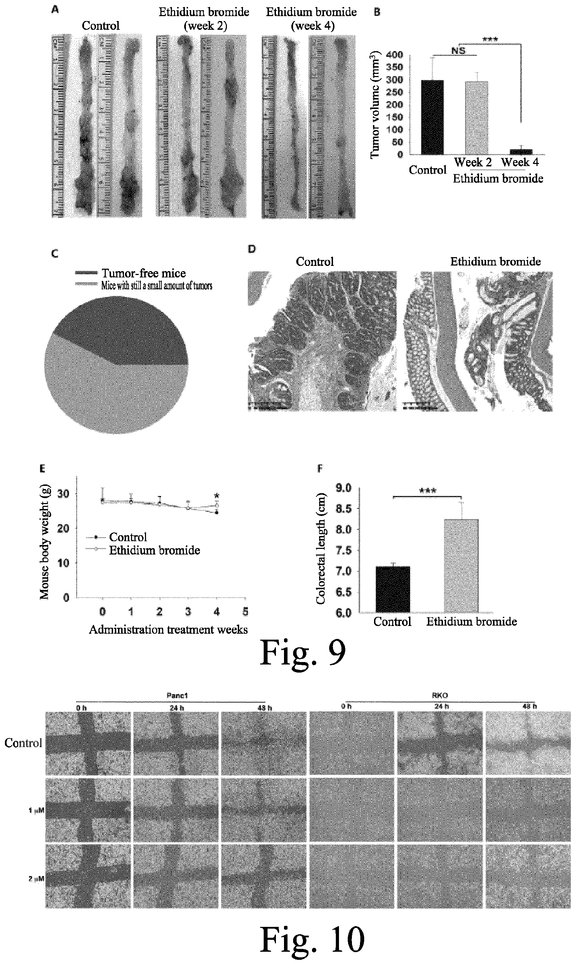

FIG. 9 shows the anti-tumor effect of ethidium bromide on advanced tumors (drug treatment started approximately 4 weeks after induction was completed). (A) Colorectal tumor status in mice of the control group, and after 2 and 4 weeks of the dosing group, respectively. (B) Tumor volumes in different treatment groups. (C) Proportion of mice with complete tumor clearance by ethidium bromide. (D) H & E staining of colorectal samples of mice in the dosing and control groups. (E) Body weight of mice in the dosing and the control groups. (F) Colorectal length of mice in the dosing and the control groups.
FIG. 10 shows the inhibitory effect of ethidium bromide on the migration of pancreatic tumor cells Panc1 and colorectal cancer cells RKO *in vitro.*

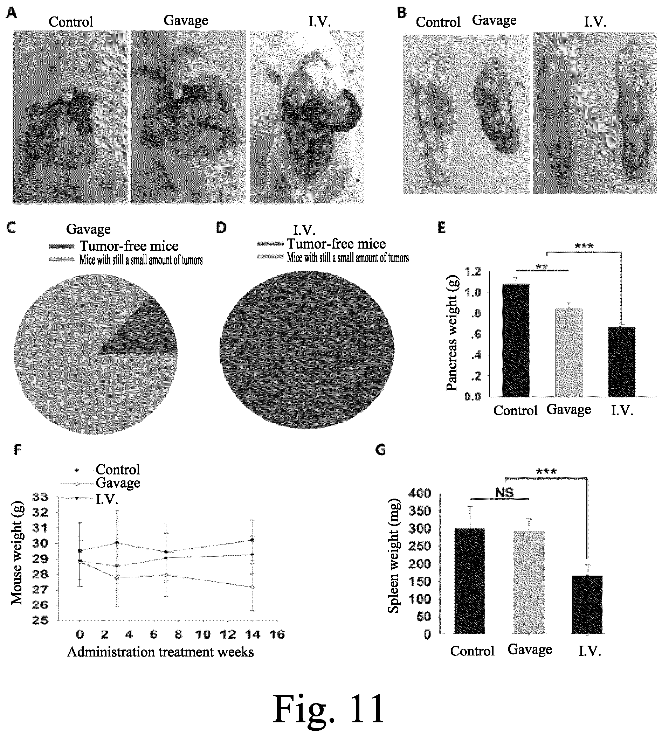

FIG. 11 shows the effect of gavage and intravenous injection of ethidium bromide on the occurrence and development of pancreatic cancer (intraperitoneal injection of 2×10⁶ Panc1) in nude mice *in vivo.* (A) Various tumor tissues in the abdominal cavity of nude mice in different treatment groups. (B) Pancreatic tumors of nude mice in different treatment control (no treatment), gavage, and intravenous administration groups. (C) and (D) Proportion of mice with complete tumor clearance when gavage and intravenously injection of ethidium bromide respectively. (E) Pancreas weights of nude mice in different treatment groups. (F) Changes in body weights of nude mice in different treatment groups. (G) Spleen weights of nude mice in different treatment groups.

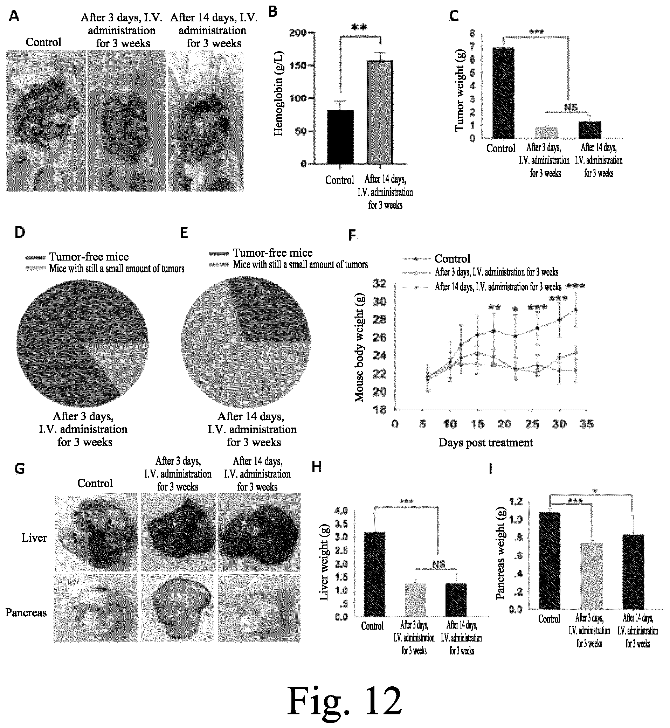

FIG. 12 shows the inhibitory effect of administration at different time points on the proliferation and migration of pancreatic cancer tumors in nude mice. (A) Tumor growth of each tissue in the abdominal cavity of nude mice. (B) The amounts of hemoglobin in the blood of nude mice in the control and the dosing groups. (C) Tumor weights of nude mice in each group. (D) Proportion of nude mice with complete tumor clearance 3 or 14 days after injection of tumor cells (E) and then 3 weeks after treatment with ethidium bromide. (F) Body weight growth curve of nude mice in each group. (G) Representative liver and pancreas morphology of nude mice in each group. (H) Statistics of liver tissue weights of nude mice in each group. (I) Statistics of pancreatic tissue weights of nude mice in each group.

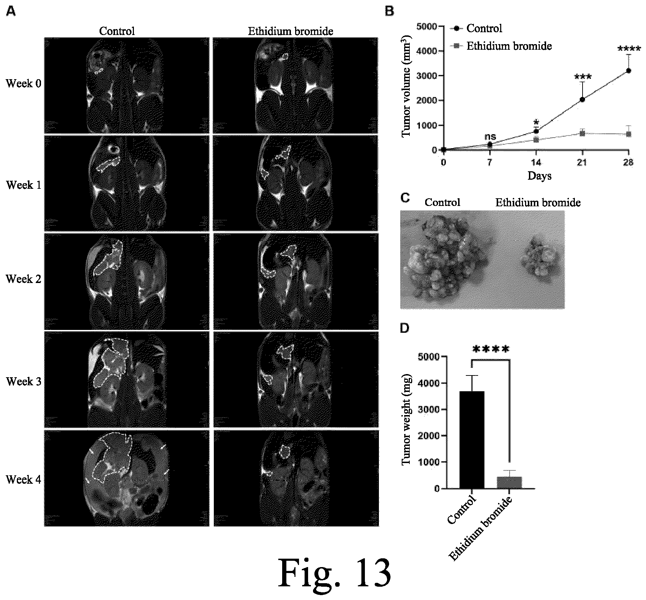

FIG. 13 shows the inhibitory effect of administration at different times on the proliferation and migration of pancreatic cancer tumors in nude mice detected by the MRI method. (A) Tumor growth of each tissue in the abdominal cavity of nude mice at different administration times detected by MRI. (the dotted line indicates the position of the tumor in the abdominal cavity, and the arrow shows the filling of ascites) (B) Growth curves of the tumors in the control and the dosing groups. (C) Abdominal cavity tumors in the control and the dosing groups removed at the end of the administration cycle. (D) Comparison of abdominal tumor weights between the control and the dosing groups at the end of the administration cycle.

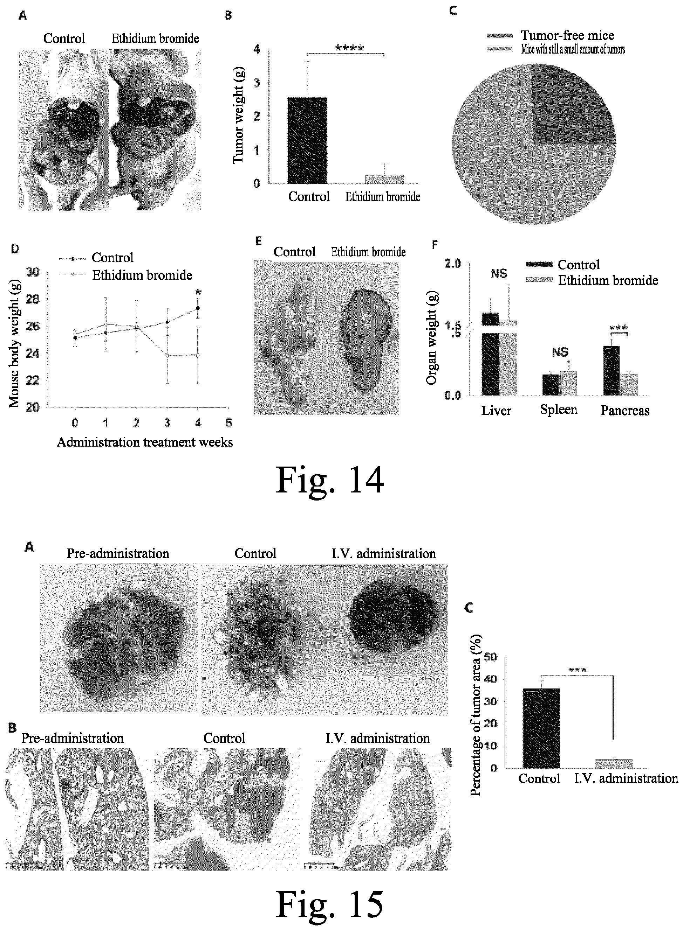

FIG. 14 shows the inhibitory effect of ethidium bromide on the proliferation and migration of colorectal cancer tumors in nude mice. (A) Tumor status in the abdominal cavity of nude mice in the control and the dosing groups. (B) Tumor weight of nude mice in the control and the dosing groups. (C) Proportion of nude mice in the dosing group with complete tumor clearance after treatment with ethidium bromide for 4 weeks. (D) Body weight growth curve of nude mice in each group. (E) Growth of pancreatic tumors in the control and the dosing groups. (F) Liver, spleen, and pancreas weights of nude mice in each group.
FIG. 15 shows the inhibitory effect of ethidium bromide on pancreatic cancer tumors that have ectopically metastasized to the lungs. (A) Tumor growth in lung tissue of nude mice approximately 3 weeks after intravenous injection of pancreatic cancer cells Panc1, i.e., pre-administration; and tumor growth in lung tissue of the control and the dosing groups 4 weeks after administration (dotted line representing the outline of tumor). (B) H & E staining of lung tumors of nude mice in different groups. (C) Proportion of lung tumor area to total lung area of nude mice in different groups.

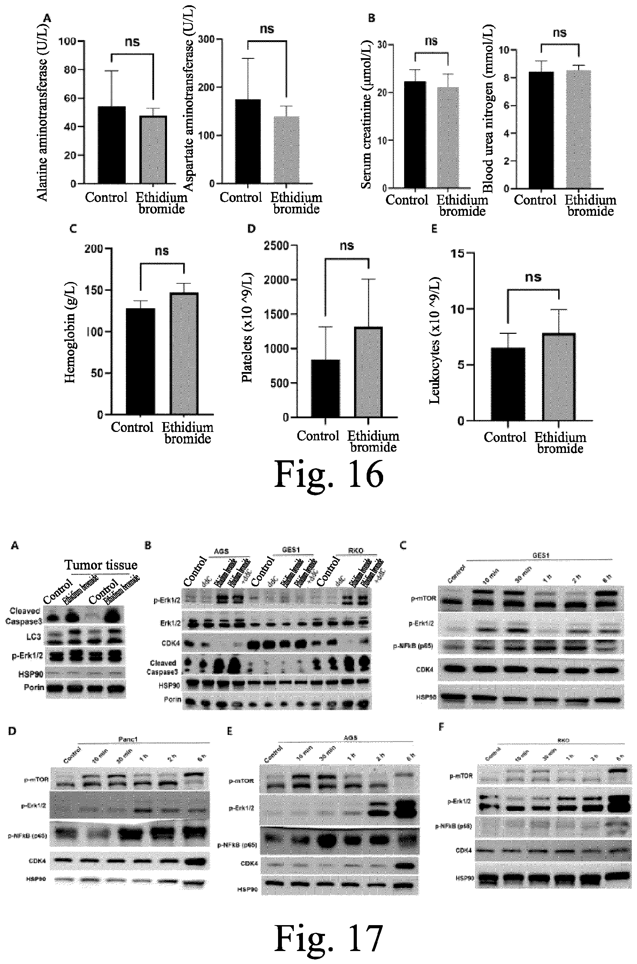

FIG. 16 shows the toxicity assay of ethidium bromide on nude mice. (A) Test of liver function in serum of nude mice. (B) Test of renal function in serum of nude mice. (C) Hemoglobin count in whole blood of nude mice. (D) Platelet count in whole blood of nude mice. (E) Leukocyte count in whole blood of nude mice.
FIG. 16 shows the effect of ethidium bromide on relevant signaling molecules in tumor tissues and cells. (A) Changes of cleaved Caspase3, p-Erk, and LC3 in tumor tissues of nude mice after 4 weeks of treatment with ethidium bromide. (B) Changes in AGS, GES1, RKO intracellular cleaved Caspase3, p-Erk, and LC3 after 3 days of treatment with ethidium bromide. Changes of relevant signaling molecules in GES1 (C), Panel (D), AGS (E), and RKO (F) cells after treatment with ethidium bromide at different times.
FIG. 17 shows the effect of ethidium bromide on relevant signaling molecules in tumor tissues and cells. (A) Changes of cleaved Caspase3, p-Erk, and LC3 in tumor tissues of nude mice after 4 weeks of treatment with ethidium bromide. (B) Changes in AGS, GES1, RKO intracellular cleaved Caspase3, p-Erk, and LC3 after 3 days of treatment with ethidium bromide. Changes of relevant signaling molecules in GES1 (C), Panel (D), AGS (E), and RKO (F) cells after treatment with ethidium bromide at different times.

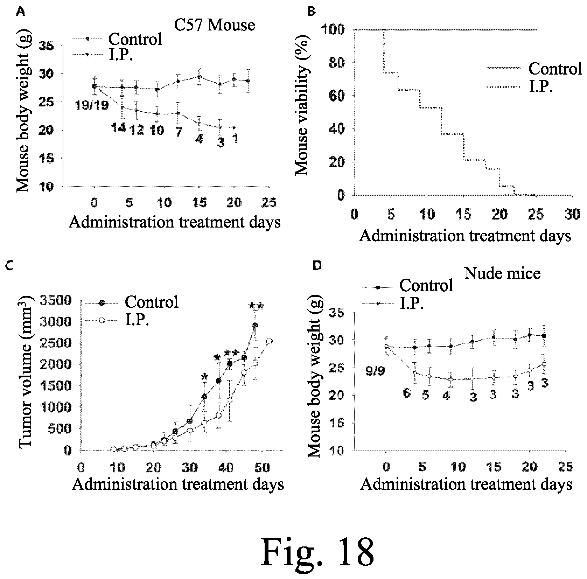

FIG. 18 shows the toxicity and tumor growth inhibitory effects of ethidium bromide administered intraperitoneally to mice. (A) Effect of different administration treatments on body weight of mice. (B) Survival curves of mice in different treatment groups. (C) Growth of subcutaneous tumors in mice. (D) Effect of different administration treatments on body weight of nude mice.

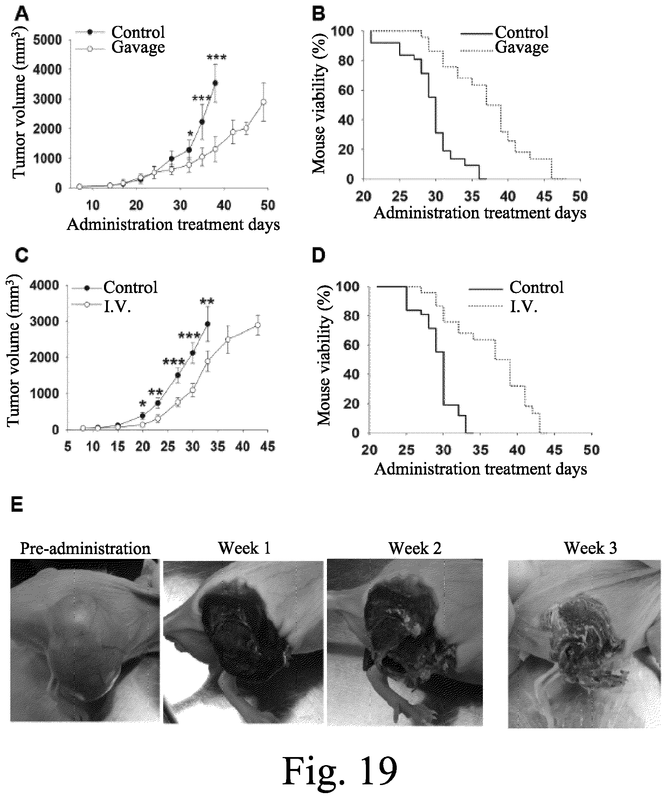

FIG. 19 shows the inhibitory effect of ethidium bromide on subcutaneous solid tumors. (A) Subcutaneous tumor growth and (B) viability in gavage-administered and control mice after subcutaneous injection of the human-derived colorectal cancer cell line RKO. Tumor growth curve (C) of and survival curve (D) of mice in the intravenously administered group and control group with the nude mice being grafted subcutaneously (PDX) with colorectal cancer tissue isolated from patients with colorectal cancer. (E) Subcutaneous tumors of PDX-molded mice at different time points after injection of ethidium bromide directly into tumors 3 times (once a day).

### Detailed Description of the Invention

### Definition

"Pharmaceutical composition" is a preparation containing a compound of the present invention in a form suitable for administration to a subject.

"Pharmaceutically acceptable" refers to those compounds, materials, compositions, carriers, and/or dosage forms which, within sound medical judgment, are suitable for use in contact with the tissues of humans and animals without excessive toxicity, irritation, allergic reactions or other problems or complications, commensurate with a reasonable benefit/risk ratio.

As used herein, "pharmaceutically acceptable salts" refers to the derivative of the compound of the present invention wherein the parent compound is modified by preparing an acid salt or base salt thereof. Examples of pharmaceutically acceptable salts include, but are not limited to, inorganic or organic acid salts of basic residues (e.g., amines), base salt or organic salts of acidic residues (e.g., carboxylic acids), and the like. For the ethidium bromide of the present invention, pharmaceutically acceptable salts include ethidium bromide, such as conventional non-toxic salts that form from non-toxic inorganic or organic acids. For example, such conventional non-toxic salts include, but are not limited to, those obtained from inorganic and organic acids selected from the group consisting of: 2-acetoxybenzoic acid, 2-hydroxyethanesulfonic acid, acetic acid, ascorbic acid, benzenesulfonic acid, benzoic acid, acid carbonic acid, carbonic acid, citric acid, ethylenediaminetetraacetic acid, ethanedisulfonic acid, 1,2-ethanesulfonic acid, fumaric acid, glucoheptonic acid, gluconic acid, glutamic acid, glycolic acid, hexylresorcinolic acid, hydrobromic acid, hydrochloric acid, hydroiodic acid, hydroxymaleic acid, hydroxynaphthoic acid, hydroxyethanesulfonic acid, lactic acid, lactobionic acid, laurylsulfonic acid, maleic acid, malic acid, mandelic acid, methanesulfonic acid, naphthalenesulfonic acid, nitric acid, oxalic acid, pamoic acid, pantothenic acid, phenylacetic acid, phosphoric acid, polygalacturonic acid, propionic acid, salicylic acid, stearic acid, basic acetic acid, succinic acid, sulfamic acid, sulfanilic acid, sulfuric acid, tannic acid, tartaric acid, toluenesulfonic acid and the like.

The compound of the present invention can exist in a solvated or non-solvated form together with other solvent molecules. "Solvate" refers to a solvent addition form containing stoichiometric or non-stoichiometric amounts of solvent. Some compounds or salts have a tendency to trap a fixed molar ratio of solvent molecules in the crystalline solid state, resulting in the formation of the solvate. If the solvent is water, the solvates formed are hydrates; if the solvent is an alcohol, the solvates formed are alcoholates. Hydrates are formed from the combination of one or more molecules of water with one molecule of a substance in which the water retains its H₂O molecular state.

The term "carrier" encompasses carriers and excipients and means a material, composition, or vehicle, such as a liquid or solid filler, diluent, excipient, solvent, or encapsulating material, involved in carrying or transporting a pharmaceutical agent from one organ, or portion of the body, to another organ, or portion of the body of a subject. Carriers include solvents, dispersion media, surfactants, antioxidants, preservatives (e.g., antibacterial agents, antifungal agents), isotonic agents, salts, preservatives, drug stabilizers, buffers (e.g., maleic acid, tartaric acid, lactic acid, citric acid, acetic acid, sodium bicarbonate, sodium phosphate, and the like), and the like, and combinations thereof, which are generally recognized as safe. Unless any conventional carrier is incompatible with the active ingredient, its use in a therapeutic or pharmaceutical composition is contemplated. Excipients include any material that acts as a carrier for the release of the active ingredient into the body of the patient and any material that, for example, is added to the active ingredient to improve its handling or to allow the resulting composition to be formed into an orally deliverable unit dose having the desired shape and consistency. Excipients can include but are not limited to, for example, diluents, disintegrants, binders, adhesives, wetting agents, lubricants, glidants, substances that mask or counteract an undesirable taste or odor, flavoring agents, dyes, substances that improve the appearance of the dosage form, and any other substances besides the active ingredients commonly used in preparing oral dosage forms.

The pharmaceutical composition of the present invention is prepared to be compatible with its intended route of administration. Examples of routes of administration include parenteral, e.g., intravenously, intra-arterially, intradermally, subcutaneously, orally (e.g., by inhalation), transdermally (topically), and transmucosally. Suitable pharmaceutical compositions for injectable use include sterile aqueous solutions (where water soluble) or dispersions and sterile powders for ad hoc preparation of sterile injectable solutions or dispersions. For intravenous administration, suitable carriers include saline, bacteriostatic water, or phosphate buffer saline (PBS). In all cases, said compositions must be sterile and should be fluid to such an extent that easy injectability exists. Also included are antibacterial and antifungal agents, e.g., parabens, tert-butyl trichloride, phenol, ascorbic acid, thimerosal, etc.; antioxidants, e.g., ascorbic acid or sodium bisulfite; chelating agents, e.g., ethylenediaminetetraacetic acid; buffers, e.g., acetates, citrates, or phosphates, and isotonic agents, e.g., sugar, polyols (e.g., mannitol, sorbitol), sodium chloride. The pH can be adjusted with an acid or base (e.g., hydrochloric acid or sodium hydroxide).

Sterile injectable solutions may be prepared by combining the active compound in the desired amount in a suitable solvent with one of the above-listed components or a combination thereof, as desired, and subsequently sterilized by filtration. Typically, dispersions are prepared by incorporating active compounds into sterile carriers containing a base dispersion medium and other required components from those listed above. In the case of sterile powder used for preparing sterile injectable solutions, the preparation method includes vacuum drying and freeze-drying, which yield powder containing active ingredients plus any additional required ingredients from the previously sterile-filtered solution. They may be enclosed in ampoules, disposable syringes, or multiple-dose vials made of glass or plastic.

Oral compositions typically include inert diluents or pharmaceutically acceptable carriers that are edible. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally, swished, and expectorated or swallowed. Pharmaceutically compatible binders, and/or adjuvant materials can be included as part of the composition. Tablets, pills, capsules, troches, and the like may contain any of the following ingredients, or compounds of a similar nature: a binder, e.g., microcrystalline cellulose, gum tragacanth, or gelatin; a diluent, e.g., starch or lactose; a disintegrant, e.g., alginic acid or cornstarch; a lubricant, e.g., magnesium stearate; a glidant, e.g., colloidal silicon dioxide; a sweetening agent, e.g., sucrose or saccharin; or a flavoring agent, e.g., menthol, methyl salicylate, or an orange flavoring agent.

The pharmaceutical composition can be included in the container, packaging, or dispenser along with the instructions for administration.

The pharmaceutical compositions of the present invention may be in bulk or unit dosage form. The unit dosage form is in a variety of forms including, for example, any of capsules, injections, IV bags, tablets, single pumps on aerosol inhalers, or vials. The amount of active ingredient (e.g., the disclosed formulation of ethidium bromide or a pharmaceutically acceptable salt, solvate, or prodrug thereof) in a unit-dose composition is the effective amount and varies according to the particular therapy involved.

It is particularly advantageous to formulate oral or parenteral compositions in the form of dosage units in order to facilitate the administration and homogeneity of the dosage. As used herein, the dosage unit form refers to a physically dispersed unit suitable for use as a unit dose for a subject to be treated; each unit contains a predetermined amount of active compound calculated to produce the desired therapeutic effect in association with the desired drug carrier. The specification for the dosage unit forms of the present invention is dictated by and directly dependent on the unique characteristics of the active compound and the particular therapeutic effect to be achieved.

"Therapeutically effective amount" refers to an amount of an agent used to treat, alleviate, or prevent an identified disease or condition, or to exhibit a detectable therapeutic or inhibitory effect. Said effect can be detected by any assay method known in the art. The precise effective amount to be used for a subject will depend on the subject's weight, size, and health; the nature and extent of the disease condition; and a therapeutic agent or combination of therapeutic agents selected for administration. A therapeutically effective amount for a given situation can be determined by routine experimentation that is within the skill and judgment of the clinician.

For any compound, the therapeutically effective amount can be estimated initially either in cell culture assays (e.g., of neoplastic cells), or in animal models (typically rats, mice, rabbits, dogs, or pigs). Animal models can also be used to determine appropriate concentration ranges and routes of administration. Such information can subsequently be used to determine available dosages and routes of administration in humans. Therapeutic/prophylactic efficacy and toxicity can be determined by standard pharmaceutical procedures in cell cultures or experimental animals, e.g. ED₅₀ (the dosage that is therapeutically effective in 50% of the population) and LD₅₀ (the dosage that is lethal in 50% of the population). The dosage ratio between toxic and therapeutic effects is the therapeutic index, which can be expressed as the ratio: LD₅₀/ED₅₀. Pharmaceutical compositions exhibiting a large therapeutic index are preferred.

The dosage may also vary within this range depending upon the dosage form employed, the sensitivity of the patient, and the route of administration. Dosage and administration are adjusted to provide sufficient levels of the active agent or to maintain the desired effect. Factors that may be considered include the severity of the disease condition, the general health of the subject, age, weight, and sex of the subject, diet, time and frequency of administration, drug combinations, reaction sensitivities, and tolerance/response to therapy. Depending on the half-life and clearance of a particular formulation, a long-acting pharmaceutical composition may be administered every 3 to 4 days, weekly or biweekly.

The dosage will also depend on the route of administration. Various routes are contemplated, including oral, pulmonary, rectal, parenteral, transdermal, subcutaneous, intravenous, intra-arterial, intramuscular, intraperitoneal, inhalation, buccal, sublingual, intrapleural, intrathecal, intranasal, and on the like. In an embodiment, the active compound is mixed under sterile conditions with a pharmaceutically acceptable carrier, as well as with any preservative, buffer, or propellant desired.

In therapeutic applications, the dosage of the pharmaceutical compositions used in accordance with the present invention varies according to the agent, the age, weight, and clinical status of the recipient patient, and the experience and judgment of the clinician or practitioner administering the therapy, in addition to other factors affecting the selected dosage. Typically, said dosage should be sufficient to result in slowing down and preferably regressing the tumor growth and also preferably causing complete regression of the cancer. The dosage may range from about 0.01 mg/kg/day to about 5000 mg/kg/day. The effective amount of an agent is the amount that provides an objectively identifiable improvement as noted by a clinician or other qualified observer. For example, regression of a tumor in a subject can be measured with reference to tumor diameter. A decrease in tumor diameter indicates regression. Regression is also indicated by the inability of the tumor to recur after cessation of treatment.

The term "administration", as used herein, refers to either directly administering a disclosed compound or a pharmaceutically acceptable salt or composition of the disclosed compound to a subject, or administering a prodrug, derivative, or analog of said compound or the pharmaceutically acceptable salt or composition of said compound to a subject, which can form an equivalent amount of the active compound in the body of the subject.

A "subject" is a cell, tissue, organ, or animal, e.g., a mammal, such as a human, mouse, rat, guinea pig, dog, cat, horse, cow, pig, or non-human primate, such as a monkey, chimpanzee, baboon, or rhesus monkey.

The term "treatment" with respect to a subject refers to ameliorating at least one symptom of the subject's condition. Treatment includes curing, ameliorating or at least partially alleviating the condition.

The methods provided herein are used to treat or alleviate symptoms of cancer. The term "cancer" includes solid tumors as well as hematologic and/or malignant tumors. A "pre-cancerous cell" or "precancerous cell" refers to a cell that exhibits a proliferative cellular disorder that is precancerous or a precursor to cancer. A "cancer cell" or "cancerous cell" is a cell that exhibits a proliferative disorder that is characterized by cancer. Any reproducible measurement can be used to identify cancer cells or precancerous cells. Cancer cells or precancerous cells can be identified by histological classification or grading of a tissue sample (e.g., a biopsy sample). Cancer cells or precancerous cells can be identified by using appropriate molecular markers. Exemplary cancers include, but are not limited to, adrenocortical cancer, AIDS-related cancer, AIDS-related lymphoma, anal cancer, anorectal cancer, anal canal cancer, appendiceal cancer, childhood cerebellar astrocytoma, childhood cerebral astrocytoma, basal cell carcinoma, skin cancer (non-melanoma), gallbladder cancer, extrahepatic cholangiocarcinoma, intrahepatic cholangiocarcinoma, bladder cancer, bone and joint cancers, osteosarcoma and malignant fibrous histiocytoma, brain cancer, brain tumor, brain stem glioma, cerebellar astrocytoma, cerebral astrocytoma/malignant glioma, ependymoma, medulloblastoma, supratentorial primitive neuroectodermal tumor, visual pathway and hypothalamic gliomas, breast cancer, bronchial adenoma/carcinoma, carcinoid tumor, gastrointestinal, nervous system cancer, nervous system lymphoma, central nervous system cancer, central nervous system lymphoma, cervical cancer, cancer of childhood stage, chronic lymphocytic leukemia, chronic myeloid leukemia, chronic myeloproliferative disease, colon cancer, colorectal cancer, cutaneous T-cell lymphoma, lymphoid paraphyte, mycosis fungoides, endometrial cancer, esophageal cancer, extracranial blastoma, extragonadal blastoma, extrahepatic cholangiocarcinoma, ocular cancer, intraocular melanoma, retinoblastoma, gallbladder cancer, gastric cancer, gastrointestinal carcinoid tumor, gastrointestinal stromal tumor, germinoma, germinal tumor of ovary, gestational trophoblastic tumor glioma, head and neck cancer, Hodgkin's lymphoma, hypopharyngeal cancer, intraocular melanoma, ocular cancer, islet cell tumor (endocrine pancreas), Kaposi's sarcoma, renal cancer, laryngeal cancer, acute lymphoblastic leukemia, acute myeloid leukemia, chronic lymphocytic leukemia, chronic myelogenous leukemia, hairy cell leukemia, lip and oral cavity cancer, liver cancer, lung cancer, non-small cell lung cancer, small cell lung cancer, AIDS-related lymphoma, non-Hodgkin's lymphoma, primary central nervous system lymphoma, Waldenström's macroglobulinemia, medulloblastoma, melanoma, Merkel cell carcinoma, malignant mesothelioma, mesothelioma, metastatic squamous cervical carcinoma, oral cancer, tongue cancer, multiple endocrine neoplasia, mycosis fungoides, myelodysplastic syndrome, myelodysplastic/myeloproliferative disorder, chronic myeloid leukemia, acute myeloid leukemia, multiple myeloma, chronic myeloproliferative disease, nasopharyngeal cancer, neuroblastoma, oropharyngeal cancer, ovarian cancer, epithelial ovarian cancer, ovarian tumor of low malignant potential, pancreatic cancer, islet cell pancreatic cancer, paranasal sinus and nasal cavity cancer, parathyroid carcinoma, penile cancer, pharyngeal cancer, pheochromocytoma, pinealocytoblastoma and supratentorial primitive neuroectodermal tumor, pituitary tumor, plasma cell paraphyte/multiple myeloma, pleuropulmonaryblastoma, prostatic cancer, rectal cancer, transitional-cell carcinoma of renal pelvic and ureteric, retinoblastoma, rhabdomyosarcoma, salivary gland cancer, sarcomatoid tumors of Ewing's family, Kaposi's sarcoma, soft-tissue sarcoma, uterine carcinoma, uterine sarcoma, skin cancer (non-melanoma), skin cancer (melanoma), Merkel cell skin cancer, small intestine cancer, soft tissue sarcoma, squamous cell carcinoma, gastric (stomach) cancer, supratentorial primitive neuroectodermal tumor, testicular cancer, laryngeal cancer, thymoma, thymoma and thymic cancer, thyroid cancer, transitional cell carcinoma of the renal pelvis, ureter, and other urinary organs, gestational trophoblastic tumor, urethral cancer, endometrial cancer, uterine sarcoma, corpus uteri cancer, vaginal cancer, vulvar cancer and Wilms' tumor.

The compounds or pharmaceutical compositions of the present invention can be administered to a subject in a number of well-known methods currently used for chemotherapeutic treatment. For example, to treat cancer, the compounds of the present invention can be injected directly into a tumor, into the blood stream or into a body cavity, or administered orally or through the skin using a patch. The dosage selected should be sufficient to result in effective treatment, but not so high as to cause unacceptable side effects. It should be preferred that the status of the disease condition (e.g., cancer, pre-cancer, etc.) and the health of the patient be closely monitored during and for a reasonable period of time after treatment.

### Description of Embodiments

In one aspect, provided by the present invention is a pharmaceutical composition comprising a therapeutically effective amount of ethidium bromide, or a pharmaceutically acceptable salt, hydrate, solvate, or prodrug thereof, and a pharmaceutically acceptable carrier.

In an embodiment, said pharmaceutical composition is for use in the treatment of cancer.

In another embodiment, said pharmaceutical composition selectively induces apoptosis in cancer cells or inhibits tumor metastasis.

Experiments have confirmed that ethidium bromide selectively induces apoptosis in cancer cells derived from a variety of different tissues. In contrast, ethidium bromide, although having an inhibitory effect on the growth of normal cells, cannot induce apoptosis in normal cells. This selectivity for specific killing of cancer cells is very valuable for potential clinical applications of ethidium bromide.

However, ethidium bromide plays a role in inhibiting tumor metastasis at different stages of tumor progression or even at advanced stages, which not only avoids further deterioration of cancer but also produces reversible or even eliminating effects on cancer progression, which will have a significant impact on survival and is of great clinical value.

Further experiments showed that ethidium bromide can selectively inhibit the mitochondrial function of cancer cells, indicating that ethidium bromide can selectively induce apoptosis in cancer cells by inhibiting mitochondrial function. This further demonstrates that defective mitochondrial function in cancerous cells plays a critical role in the selective induction of apoptosis in cancerous cells by ethidium bromide, and also suggests that mitochondria may play a unique and very important role both in the process of cancer development and in cancer treatment.

In another embodiment, said cancer is selected from one or more of gastric cancer, liver cancer, colorectal cancer, breast cancer, lung cancer, pancreatic cancer, leukemia, preferably colorectal cancer or pancreatic cancer.

Ethidium bromide has a good inhibitory effect against a variety of cancers or cancer cells, including but not limited to several verified by the present invention. Especially for pancreatic cancer, which is recognized to be highly malignant, rapidly progressive, poorly treated, and with a short survival period, ethidium bromide still has excellent inhibitory effects, including inhibition of cancer metastasis and induction of apoptosis in cancer cells.

In another embodiment, said pharmaceutical composition is in oral or injectable form.

The traditional way of administration by intraperitoneal injection (I.P.) not only has excessive side effects but also has a relatively poor anti-tumor effect, which may be one of the reasons why ethidium bromide has not been used for effective anti-tumor use in the prior art to date. In contrast, the present invention has for the first time found that ethidium bromide administered orally or by injection, in particular intravenous injection (I.V), has a superior anti-tumor effect and is less toxic. Moreover, this anti-tumor effect targets *in vivo* tumor tissues, which is closer to the *in vivo* pathogenesis of cancer than studies targeting subcutaneous solid tumors, with better prospects for medical applications and greater value.

In another aspect, provided by the present invention is use of ethidium bromide, or a pharmaceutically acceptable salt, hydrate, solvate, or prodrug thereof, in the preparation of the aforementioned pharmaceutical composition.

In another aspect, provided by the present invention is a method for treating cancer comprising administering to a subject in need thereof the aforementioned pharmaceutical composition.

In yet another aspect, provided by the present invention is a method for inhibiting tumor metastasis, comprising administering to a subject in need thereof the aforementioned pharmaceutical composition.

In yet another aspect, provided by the present invention is a method for activating the mTOR signaling pathway comprising administering to a subject in need thereof the aforementioned pharmaceutical composition.

Experiments have shown that ethidium bromide can rapidly activate mTOR, followed by rapid induction of downstream NFkB and Erk phosphorylation and Caspase3 cleavage, and the activation of these signaling molecules ultimately and selectively induced tumor cell apoptosis. This early event that induces apoptosis in tumor cells can occur both *in vitro* and be reproduced *in vivo.*

In one embodiment, said administration is performed orally, by injection, parenterally, subcutaneously, or by infusion, preferably orally or by injection, particularly preferably by injection, e.g., intravenously, intra-arterially, subcutaneously, or intramuscularly.

In another embodiment, the administration is performed at a dosage of from about 1 mg/Kg to 100 mg/Kg of body weight per day, e.g., about 1 mg/Kg, about 2 mg/Kg, about 5 mg/Kg, about 10 mg/Kg, about 15 mg/Kg, about 20 mg/Kg, about 25 mg/Kg, about 30 mg/Kg, about 35 mg/Kg, about 40mg/Kg, about 50 mg/Kg, about 60 mg/Kg, about 70 mg/Kg, about 80 mg/Kg, about 90 mg/Kg, about 100 mg/Kg of body weight per day.

In general, said dosage should be sufficient to cause slowing and preferably regress the tumor growth and also preferably cause complete regression of the tumor. The dosage may for example range from about 0.01 mg/kg/day to about 5000 mg/kg/day.

When mice were treated in the present invention by conventional intraperitoneal administration, the dosage used was 10 mg/Kg, but most of the mice were very sensitive and died excessively. Gavage oral administration of 20 mg/Kg ethidium bromide per day was well tolerated and had better anti-tumor effects in mice. The intravenous dosage was 5 mg/Kg per day for 4 weeks. Intravenous administration has proved to have the lowest therapeutically effective dose, the least toxic side effects, and the best anti-tumor effect.

The specific ethidium bromide dosage will vary according to the dosage form employed, the sensitivity of the patient and the route of administration, and the experience and judgment of the clinician or practitioner administering the therapy.

In another embodiment, the administration is continuously performed for at least 3 days, at least 4 days, at least 5 days, at least 6 days, at least 1 week, at least 2 weeks, at least 3 weeks, at least 4 weeks, at least 2 months, at least 3 months, at least 4 months, at least 5 months, at least 6 months, or at least 1 year.

After 3-4 weeks of continuous administration of ethidium bromide of the present invention, tumors regressed significantly or even completely. The specific period of administration will vary according to the dosage form employed, the sensitivity of the patient and the route of administration, and the experience and judgment of the clinician or practitioner administering the therapy. For example, for advanced tumors, the period of administration needs to be longer.

In another embodiment, the administration is performed for one or more (e.g., 1, 2, 3, 4, 5, 6, 7, 8, 9, or 10) courses of treatment, each course of treatment lasts at least 3 days, at least 4 days, at least 5 days, at least 6 days, at least 1 week, at least 2 weeks, at least 3 weeks, or at least 4 weeks; and wherein there is an interval of 0, 1, 2, 3, 4, 5, 6, 7, 8, 9, 10 days, two weeks, three weeks, or four weeks between every two courses of treatment.

After one continuous course of continuous administration of ethidium bromide of the present invention, tumors regressed significantly or even completely. The specific course of administration will vary according to the dosage form employed, the sensitivity of the patient and the route of administration, and the experience and judgment of the clinician or practitioner administering the therapy.

In yet another aspect, provided by the present invention is a method for selectively inducing apoptosis in cancer cells comprising contacting said cancer cell with ethidium bromide, or a pharmaceutically acceptable salt, hydrate, solvate, or prodrug thereof.

This event of selective induction of apoptosis in cancer cells can occur both *in vitro* and be reproduced *in vivo.* In an embodiment, said cancer cells are derived from one or more of gastric cancer, liver cancer, colorectal cancer, breast cancer, lung cancer, pancreatic cancer, leukemia, preferably colorectal cancer or pancreatic cancer.

### Examples

The present invention is further illustrated by the following specific examples. It should be understood that these examples are only used to illustrate the present invention and not to limit the scope of the present invention. Furthermore, it will be appreciated that those skilled in the art, upon reading the teachings of the present invention, may make various changes and modifications to the present invention and that such equivalents are intended to fall within the scope of the appended claims of the present invention.

### Example 1

Ethidium bromide selectively inducing tumor cell death

### 1. Ethidium bromide selectively inducing tumor cell apoptosis

To verify the important role of mitochondrial function in tumor treatment and occurrence, cancerous and non-cancerous cells were treated with the mitochondrial damage-inducing reagent, ethidium bromide, in this Example.

Ethidium bromide was purchased from Sigma (E7637) and treated as follows. Cancer and normal cells of various tissue origins with approximately 40% growth abundance were treated with 0.1, 0.4, and 1 µg/ml for 48 h, followed by PI staining and cycle detection by flow cytometry, statistical analysis of genomic DNA break (Sub-G1) cells, or cell viability by MTT method.

The results showed that ethidium bromide can selectively induce the death of a wide range of cancer cells, including hepatocellular carcinoma cells (SK-Hep1, BEL-7404), and melanoma cells (B16), whereas for normal cells, such as hepatocellular cell lines (Chang, LO2), and fibroblast cell lines (3T3-L1), it inhibited growth at most and cannot induce apoptosis (FIG. 1).

In support of this, cancer cells and normal cells from various tissues with growth abundance of about 40% were treated with 1, 2, and 5 µM for 48 h, respectively, and then observed the growth of the cells by light microscopy, followed by PI staining, detection and statistical analysis of genomic DNA break (Sub-G1) cells by flow cytometry (FIG. 3), or cell apoptosis detection by Annexin V staining method (FIG. 4).

The results showed that ethidium bromide can selectively induce the death of cancer cells in many different tissues, including hepatocellular carcinoma cells (SK-Hep1, 7404), gastric cancer cells (AGS), colorectal cancer cells (RKO), whereas for normal cells such as normal hepatocellular cell line (LO2, chang), gastric epithelial cell line (GES1), it only inhibited growth and cannot induce apoptosis (FIGs. 2-4).

In support of this, Table 1 summarizes the median lethal dosage of ethidium bromide on tumor cells and normal cells from various sources, and the data indicate that the vast majority of median lethal dosages of ethidium bromide against these tumor cells are below 10 µM, whereas those against normal cells are mostly above 50 µM.

**Table 1. Median lethal dosage(IC₅₀) of ethidium bromide against various tumor cell lines and normal cell lines**

| | N2A | Hela | MDA-231 | RKO | PANC1 | B16 | HepG2 | AGS | Sk-Hep1 | Nalm-6 | Reh |
|---|---|---|---|---|---|---|---|---|---|---|---|
| Tumor cells | Neurog lioma | Cervical cancer | Breast cancer | Colorec tal cancer | Pancreatic cancer | Melano ma | Liver cancer | Gastric cancer | Liver cancer | Leuke mia | Leukem ia |
| IC₅₀(µM) | 15.7 | 3.35 | 5.4 | 1.433 | 3.297 | 11.19 | 8.12 | 0.71 | 1.26 | 1.17 | 2.23 |
| | | | | | | | | | | | |
| Normal cells | MEF | GES1 | 3T3-L1 | LO2 | C2C12 | | | | | | |
| IC₅₀(µM) | 38.51 | 64.66 | 67.88 | 50.73 | 85.52 | | | | | | |

In conclusion, ethidium bromide can specifically induce cancerous cell death, and the mode of death is mainly apoptosis. However, the effect of ethidium bromide on non-cancerous cells (normal cells) is only growth inhibition and does not result in cell death. This may be related to the mechanism by which ethidium bromide induces mitochondrial damage and alters energy metabolism in tumor tissues.

### 2. Selective inhibition of mitochondrial function in cancer cells by ethidium bromide

To further verify the effect of ethidium bromide on mitochondrial function, various cell lines were treated with ethidium bromide and mitochondrial function was tested by using Seahorse after 6 hours. The results showed that the parameters reflecting mitochondrial function, such as oxygen consumption, glycolysis level, and glycolysis rate, were significantly reduced in pancreatic cancer cells Panc1 and colorectal cancer cells RKO after being treated with ethidium bromide, and manifested as a certain concentration-dependent effect (the numbers are the concentration values in µM). In contrast, the normal cell line GES1 decreased only at higher concentrations such as 5 µM, and the effective concentration of ethidium bromide toxicity was significantly higher than that of cancer cells (FIG. 5).

This indicates that ethidium bromide can selectively inhibit the mitochondrial function of cancer cells, and suggests that ethidium bromide can selectively induce apoptosis in cancer cells by inhibiting mitochondrial function. This further demonstrates that defective mitochondrial function in cancerous cells plays a critical role in the selective induction of death in cancerous cells by ethidium bromide, and also suggests that mitochondria may play a unique and very important role both in the process of cancer occurrence and in cancer treatment.

### Example 2

### Antitumor activity of ethidium bromide on tumorigenesis in mice in vivo

### 1. Colorectal cancer induced by intraperitoneal injection (spontaneous tumorigenesis)

### 1) Administration after 1 week

The classical intraperitoneal injection of AOM and the spontaneous colorectal cancer formation by three rounds of DSS were utilized to construct a tumor mouse model. Mice were treated with ethidium bromide (500 mg/L dissolved in drinking water) one week after completion of the construction. After 2 months of administration, control mice showed significant prolapse due to oversized colorectal tumors, whereas the dosing group did not (Figure 6A). Mice were sacrificed and their colorectum was taken for photographic observation and fixed staining. The results of the study showed that ethidium bromide had very good activity against colorectal tumors, with almost complete disappearance of the tumor mass in the dosing group (FIGs. 6 B and C)

To further verify the above results, we then expanded the sample size to about 10-16 mice per group and still used the above AOM+DSS protocol to induce spontaneous colorectal cancer formation in mice. Because it is very difficult to administer each mouse in drinking water, and because a significant proportion of mice do not drink drinking water containing ethidium bromide, we switched to gavage administration (20 mg/Kg once daily). Meanwhile, since it has been reported that zalcitabine (ddC) can effectively impair the function of mitochondria (mainly the replication of mtDNA, similar to ethidium bromide), we added the experimental group of zalcitabine (33 mg/Kg) as a control.

The tumors in the pre-administration mice are shown in FIG. 7A. After one month of administration, the spontaneous colorectal tumors completely disappeared in more than 80% of the ethidium bromide-treated group of mice, with very few tumor masses remaining in the colorectum of only a few mice (FIGs. 7B-E), but the administration of zalcitabine alone had no effect, which was almost the same as that of the control group. Meanwhile, the size of the spleen, length of colorectum, and weight of tissue organs were healthier in the dosing group as compared to the control group (FIGs. 7F-H), whereas the body weight was little changed (FIG. 7I).

We tested the mtDNA copy number of colorectal tumors in the dosing group and, surprisingly, ethidium bromide did not alter the mitochondrial mtDNA copy number (FIG. 7J).

In addition, in order to investigate in what way ethidium bromide clears tumors in mice, we also tested cell proliferation (FIGs. 8A and B) and apoptosis (FIG. 8C-F) in tumor tissues and normal tissues surrounding the tumors in different treatment groups. Among them, cell proliferation was detected by immunohistochemistry of Ki67-positive cells, and apoptosis was detected by immunofluorescence staining of cleaved Caspase3. The results showed that the proliferation of tumor cells in the ethidium bromide-treated group was significantly lower than that of cells in the other treatment groups, while on the contrary, Caspase3-positive tumor cells (i.e., apoptotic cells) were significantly higher than that of cells in the other groups and the surrounding normal tissues. This suggests that ethidium bromide clears tumor tissue primarily by inducing apoptosis in tumor cells.

### 2) Administration after 4 weeks

To further verify whether ethidium bromide is still effective against already existing larger tumors (advanced colorectal cancer), we set aside a group of control mice (started administration 5 weeks after the end of induction, equivalent to the control group of mice described above) to administer them by gavage (20 mg/Kg per day).

The results showed that ethidium bromide was similarly very effective for these larger tumors, with more than 40% of the mice achieving complete tumor clearance after 1 month of administration, and the remaining tumors that were not completely cleared were also very small (FIG. 9), and are expected to be completely cleared after another week or so as well.

### 2. Ectopic metastasis

### 1) in vitro detection

Ectopic metastasis of tumors is the biggest challenge in tumor treatment. In order to further investigate whether ethidium bromide can effectively inhibit the migration of tumors, we firstly tested the effect of ethidium bromide on the migration of tumor cells *in vitro.* The scratch assay of cultured cells showed that ethidium bromide can indeed significantly inhibit the migration of pancreatic tumor cells Panel and colorectal cancer cells RKO (FIG. 10).

### 2) In vivo detection

To further verify whether ethidium bromide can inhibit tumor migration *in vivo,* we injected 2 × 10⁶ pancreatic cancer cells Panc1 intraperitoneally into nude mice, which were then divided into 3 groups: control group, gavage, and intravenous injection administration. Mice in the gavage-administration group were started with gavage oral administration of 20 mg/Kg ethidium bromide per day by gavage 3 days after intraperitoneal injection of pancreatic cancer cells. Mice in the intravenous administration group were administered with 5 mg/Kg ethidium bromide per day intravenously (Tail-IV) 3 days after intraperitoneal injection of pancreatic cancer cells.

The results showed that ethidium bromide can significantly inhibit the proliferation of pancreatic cancer *in vivo,* especially in the intravenous injection group, no tumor was found in the pancreas of nude mice, indicating its very excellent anti-tumor effect. However, the pancreas of nude mice in the gavage group still had a small amount of tumor, but was significantly smaller than that in the control group (FIGs. 11A-D). Accordingly, the weight of the pancreas of the administration group, especially the intravenous injection administration group, was significantly smaller than that of the control group (FIG. 11E). This further illustrates the exciting anti-tumor effect of ethidium bromide, even against malignant pancreatic cancer cells. The results also showed that the intravenous tumors, intravenous injection administration was superior to other administration modes such as gavage administration, which was essential for the selection of subsequent administration methods.

The body weight data of nude mice showed that compared with gavage administration, intravenous injection administration did not cause a significant decrease in body weight of nude mice, indicating that intravenous injection administration is even slightly less toxic to nude mice than the other modes of administration, where the control nude mice weighed slightly more than the dosing group, partly due to the fact that ethidium bromide caused a slight decrease in the animals' body weight, and partly due to the fact that the body weights of the control mice increased with the increase of the tumor (FIG. 11F). In addition, the control and gavage administration groups (especially the control group) had significantly larger spleens than the intravenous administration group due to the fact that the pancreas contained tumors, which induced and activated the immune system (FIG. 11G). These results further highlight the superiority of intravenous administration for cancer treatment.

### Example 3

### 1. Advanced neoplasia: intraperitoneal injection of pancreatic cancer cells

In order to further investigate the anti-tumor effect of ethidium bromide on nude mice with advanced tumorigenicity and to obtain its effect on the viability of nude mice, we intraperitoneally injected the nude mice with 2 × 10⁶ pancreatic cancer cells Panc1, which were then divided into 3 groups, the control group (no-treatment group), the early-injection group (administered intravenously after 3 days) and the late-injection group (administered intravenously after 14 days). Mice in the early injection group were administered with 5 mg/Kg ethidium bromide intravenously per day for 3 weeks 3 days after intraperitoneal injection of pancreatic cancer cells (starting intravenous administration after 3 days for about 3 weeks). Mice in the late injection group were administered with 5 mg/Kg ethidium bromide intravenously per day for 3 weeks starting 2 weeks days after intraperitoneal injection of pancreatic cancer cells (starting intravenous administration after 14 days for about 3 weeks).

As the control nude mice had severe ascites due to oversized tumors during this process, the administration was stopped after 3 weeks of continuous administration. At the same time, the abdominal cavity of the control nude mice was filled with ascites; while the abdominal cavity of the dosing group was very normal (results not shown). FIG. 12A shows tumor growth of various tissues in the abdominal cavity of nude mice. The data on hemoglobin in nude mice also showed that at this time the control nude mice had a severe decrease in blood hemoglobin due to severe blood loss from ascites (which contains a large number of erythrocytes), whereas the blood hemoglobin even in the nude mice of the late dosing group remained relatively normal (FIG. 12B).

Meanwhile, the results showed that the pancreatic cancer tumor of nude mice in the dosing group was significantly smaller than that in the control group (FIG. 12C). More than 80% of the nude mice in the early tumor-injected group had no tumor (FIG. 12D), and about 45% of the nude mice in the late tumor-injected group had no tumor at all (FIG. 12E). Notably, because the nude mice had very advanced tumor metastases and large amounts of ascites production, the control nude mice showed a significant increase in body weight at the later stage, whereas the body weights of the nude mice in the dosing group were relatively stable (FIG. 12F). In addition, control nude mice were filled with tumors on the outer surface of the liver, pancreas, and small intestinal tissues. This was particularly true for metastases to liver tissue, which was almost entirely occupied by tumors in control nude mice. In contrast, even the tumors in the liver tissues of the nude mice in the late injection group were significantly reduced compared with the control group (FIG. 12G). Correspondingly, the weights of liver and pancreatic tissues were significantly smaller even in the mice of the late-injected group compared with that of the control group (FIGs. 12H and 12I). The above results further indicate that ethidium bromide has very superior anti-tumor activity in advanced malignant pancreatic cancer. In addition, we utilize the most advanced nuclear magnetic spectrometer uMR 9.4T with a 30 cm aperture magnet jointly developed by our work unit and Shanghai United Imaging Healthcare Co., LTD, as well as our self-developed GC, GPA, and VTC42 coils specially designed for abdominal scanning, and an optional PET insert system to achieve the PET-MR imaging results of the temporal and spatial integrality. The development of pancreatic cancer was followed dynamically in the control and dosing groups following intraperitoneal injection of pancreatic cancer cells. Mice were injected intraperitoneally with 2 × 10⁶ pancreatic cancer cells, Panc1, and left for 2 weeks after which a visible tumor mass can be detected in the abdominal cavity to begin intravenous administration of 5 mg/Kg ethidium bromide for 4 weeks. Similar to the above correlation results: the growth of pancreatic cancer in the abdominal cavity of the mice was significantly inhibited, or almost completely inhibited, after administration. Moreover, the abdominal cavity of the control mice was filled with ascites by the late stage (where the arrows are shown), whereas there was none in the dosing group (FIGs. 13A and 13B). We removed and photographed the tumors in the mice and showed that the MRI results were indeed very reliable (FIGs. 13C and 13D). However, clearance was not achieved mostly as in the previous results and we analyzed that it might be due to the fact that the mice were kept in a non-SPF environment during the whole administration period of MRI, thus affecting the effect of the drug.

### 2. Advanced neoplasia: intraperitoneal injection of colorectal cancer cells

In order to further investigate whether ethidium bromide also has a better inhibitory effect on other types of tumors, we administered nude mice 2 weeks after the intraperitoneal injection of 2 × 10⁶ colorectal cancer cells RKO, and then tested the anti-tumor effect of ethidium bromide after 4 weeks of intravenous administration, as described above.

The results showed that ethidium bromide still remained very effective in inhibiting colorectal cancer of human origin and its metastasis. The abdominal tumors of nude mice in the dosing group were significantly smaller than those in the control group (FIGs. 14A-B), and were mainly concentrated in the external intestinal and pancreatic sites. At the same time, tumors were completely cleared in approximately 25% of the mice in the dosing group (FIG. 14C). There was no significant difference in body weight between the two groups of mice in the early stage, and in the later stage, the control mice showed significant weight gain due to the enlargement of the tumors and also a small amount of ascites produced in the abdominal cavity of the mice, whereas the dosing group showed a slight decrease in body weight (FIG. 14D). There was no significant difference in liver and spleen weights between the two groups of mice, and the control mice became heavier because their pancreatic tissues were full of metastases (FIGs. 14E-F). It can be seen that ethidium bromide has a very good inhibitory effect on both the proliferation and metastasis of colorectal cancer cells of human origin.

### 3. Ectopic tissue metastasis in vivo

In addition, to further investigate the effect of ethidium bromide on ectopic tissue metastatic tumors, we injected 2 × 10⁶ pancreatic cancer cells Panc1 intravenously into nude mice. After ectopic tissue metastasis, tumors formed primarily in the lungs. After approximately 3 weeks, intravenous administration of 5 mg/Kg ethidium bromide per day was started after the tumor had already grown in the lung tissue (FIG. 15A). The mice were sacrificed after 4 weeks of administration and the lungs were removed for test. The results showed that the tumors in the lung tissues of the dosing group were significantly smaller than those in the control group (FIG. 15A-C), further indicating that ethidium bromide exerted a significant inhibitory effect on the proliferation of metastatic tumors in ectopic tissues.

### 4. Toxicity of ethidium bromide

Finally, we also conducted a preliminary study on the toxicity of ethidium bromide by collecting whole blood and serum from the control and the dosing groups (administered intravenously for about 1 month) and tested their routine blood, and liver and kidney functions, respectively.

The data demonstrated that after 4 weeks of intravenous administration, ethidium bromide showed no significant difference in liver or kidney function in nude mice compared with the control group (FIGs. 16A and 16B). In addition, blood routine data such as hemoglobin, platelets and leukocytes were very normal in both groups of nude mice (FIGs. 16C, 16D, and 16E), thus demonstrating that ethidium bromide exerts its superior anti-tumor function with very low toxicity. This laid the foundation for its final clinical transformation.

### 5. Potential mechanism of action of ethidium bromide

We investigated the mechanism of ethidium bromide-induced apoptosis in tumor cells. First, tumor tissues treated with ethidium bromide (mice shown in FIGs. 7 and 8, treated for 4 weeks at 20 mg/Kg per day) were subjected to SDS-Loading buffer boiling lysis and tested for associated signaling pathways by Western blotting.

The results showed that ethidium bromide treatment resulted in a significant increase in cleaved Caspase3 in tumor tissues, as well as phosphorylation of Erk and the level of LC3, an important protein for autophagy (FIG. 17A). Consistent with this, *in vitro* cells treated with ethidium bromide showed a significant increase in the phosphorylation levels of both cleaved Caspase3 and Erk (FIG. 17B).

In order to further determine how ethidium bromide activates phosphorylation of Erk and Caspase3 cleavage and induces apoptosis, we also tested with different cell lines treated with ethidium bromide for 10 min, 30 min, 1, 2, and 6 h. The results showed that mTOR was already activated by ethidium bromide very quickly at 10 min, while p-NFkB and p-Erk were also activated at 30 min and 1 h, respectively, suggesting that the activation of these signaling molecules is an early event in ethidium bromide-induced apoptosis in tumor cells.

### Example 4

### 1. Toxicity of ethidium bromide in mice by intraperitoneal injection

In order to further test whether ethidium bromide still has strong anti-tumor activity against solid tumors *in vivo,* C57 mice were first injected subcutaneously with 2 × 10⁶ mouse melanoma cells B16, and the administration was initiated after about 10 days when the subcutaneous tumor volume of the mice reached about 30 mm³. We first treated the mice by traditional intraperitoneal administration (10 mg/Kg). The results showed that the mice were very sensitive to intraperitoneal injection of ethidium bromide, and after 3 days of continuous injection, mice started to die one after another and began to lose weight significantly. So after 3 days, we switched to injecting every other day. By the end of the experiment, only 1 of the 19 mice survived (FIGs. 18A and B). Although the tumors of the mice in the dosing group were slightly smaller than those in the control group, the tumor suppression effect was not obvious (FIG. 18C). Therefore, it showed that the traditional way of intraperitoneal injection not only had excessive side effects but also had poor anti-tumor effect.

In addition, we also tested for nude mice utilizing intraperitoneal administration, where 2 × 10⁶ colorectal cancer cells, RKO, were first injected intraperitoneally in nude mice, and about 3 days later, we treated the nude mice using intraperitoneal administration (10 mg/Kg). Similar to C57 mice, once daily until day 5, administration was stopped due to excessive deaths in nude mice (FIG. 18D).

### 2. Limitation of anti-tumor activity of ethidium bromide against subcutaneous tumorigenic and subcutaneous patient colorectal cancer grafted (PDX) tumor in mice

We also tested the anti-tumor activity of ethidium bromide against subcutaneous solid tumors. 2 × 10⁶ colorectal cancer cells RKO were injected subcutaneously. About 1 week later, when solid tumors grew subcutaneously in nude mice, gavage administration (20 mg/Kg) was started. The size of the tumors was measured twice a week. It was found that although ethidium bromide exhibited some tumor proliferation inhibitory activity and also contributed to improve the viability of nude mice, the inhibitory effect of ethidium bromide on subcutaneous solid tumors was very unsatisfactory compared to its very strong inhibitory effect on tumor cell proliferation and migration *in vivo* (FIGs. 19 A and B).

In addition, we also grafted isolated tumor tissue blocks (PDX) from patients with colorectal cancer subcutaneously in nude mice and similarly found that the anti-tumor effect of ethidium bromide was very limited and much less than its tumor inhibitory effect on various tissues *in vivo* (FIG. 19 C and D).

However, when we injected ethidium bromide directly into the subcutaneous tumor mass (20 mg/Kg ethidium bromide dissolved in PBS, injected once a day for three consecutive injections), the subcutaneous tumor mass was rapidly necrotic and eventually died and fell off (FIG. 19E). Ethidium bromide also showed excellent anti-tumor activity.

Taken together, we hypothesize that the limitations of ethidium bromide for subcutaneous solid tumors may be primarily due to the difficulty of drug molecules administered *in vivo* to reach the subcutis. The value of subcutaneous neoplasia is to facilitate scientific research, but the test results of subcutaneous neoplasia may not be repeated *in vivo,* and there is great difference between subcutaneous neoplasia and *in vivo,* so it is not practical. In contrast, the present invention has a very good inhibitory effect on the proliferation and metastasis of tumor tissues *in vivo* by infusion or intravenous injection of ethidium bromide with little or almost no side effect, showing great medical application value.

## Claims

1. A pharmaceutical composition comprising a therapeutically effective amount of ethidium bromide, or a pharmaceutically acceptable salt, hydrate, solvate, or prodrug thereof, and a pharmaceutically acceptable carrier.

2. The pharmaceutical composition of claim 1 for the treatment of cancer.

3. The pharmaceutical composition of claim 2, wherein the pharmaceutical composition selectively induces apoptosis in cancer cells or inhibits tumor metastasis.

4. The pharmaceutical composition of claim 2, wherein the cancer is selected from one or more of gastric cancer, liver cancer, colorectal cancer, breast cancer, lung cancer, pancreatic cancer, and leukemia, preferably colorectal cancer or pancreatic cancer.

5. The pharmaceutical composition of any one of the preceding claims, wherein the pharmaceutical composition is in oral or injectable form.

6. Use of ethidium bromide, or a pharmaceutically acceptable salt, hydrate, solvate, or prodrug thereof, in the preparation of the pharmaceutical composition of any one of claims 1-5.

7. A method for treating cancer, comprising administering to a subject in need thereof the pharmaceutical composition of any one of claims 1-5.

8. A method for inhibiting tumor metastasis, comprising administering to a subject in need thereof the pharmaceutical composition of any one of claims 1-5.

9. A method for activating an mTOR signaling pathway, comprising administering to a subject in need thereof the pharmaceutical composition of any one of claims 1-5.

10. The method of any one of claims 7-9, wherein the administration is performed orally, by injection, parenterally, subcutaneously or by infusion, preferably orally or by injection, particularly preferably by injection, e.g., intravenously, intra-arterially, subcutaneously, or intramuscularly.

11. The method of any one of claims 7-10, wherein the administration is performed at a dosage of from about 1 mg/Kg to 100 mg/Kg of body weight per day, e.g., about 1 mg/Kg, about 2 mg/Kg, about 5 mg/Kg, about 10 mg/Kg, about 15 mg/Kg, about 20 mg/Kg, about 25 mg/Kg, about 30 mg/Kg, about 35 mg/Kg, about 40mg/Kg, about 50 mg/Kg, about 60 mg/Kg, about 70 mg/Kg, about 80 mg/Kg, about 90 mg/Kg, about 100 mg/Kg of body weight per day.

12. The method of any one of claims 7-11, wherein the administration is continuously performed for at least 3 days, at least 4 days, at least 5 days, at least 6 days, at least 1 week, at least 2 weeks, at least 3 weeks, at least 4 weeks, at least 2 months, at least 3 months, at least 4 months, at least 5 months, at least 6 months, or at least 1 year.

13. The method of any one of claims 7-12, wherein the administration is performed for one or more (e.g. 1, 2, 3, 4, 5, 6, 7, 8, 9, or 10) courses of treatment, each course of treatment lasts at least 3 days, at least 4 days, at least 5 days, at least 6 days, at least 1 week, at least 2 weeks, at least 3 weeks, or at least 4 weeks; and wherein there is an interval of 0, 1, 2, 3, 4, 5, 6, 7, 8, 9, 10 days, two weeks, three weeks, or four weeks between every two courses of treatment.

14. A method for selectively inducing apoptosis in cancer cells, comprising contacting the cancer cells with ethidium bromide, or a pharmaceutically acceptable salt, hydrate, solvate, or prodrug thereof.

15. The method of claim 14, wherein the cancer cells are derived from one or more of gastric cancer, liver cancer, colorectal cancer, breast cancer, lung cancer, pancreatic cancer, and leukemia, preferably colorectal cancer or pancreatic cancer.
